# EUROPEAN PATENT SPECIFICATION

(11) **EP 0 624 573 B1**
(45) Date of publication and mention of the grant of the patent: **15.10.1997**
(21) Application number: 94303389.4
(22) Date of filing: 11.05.1994
(51) Int. Cl.: C07C 401/00, A61K 31/59

(54) **Iodo vitamin D3 compounds and method for preparing same**
Jodvitamin-D-Verbindungen und Verfahren zu ihrer Herstellung
Composés de iodovitamine D et procédé pour leur préparation

(30) Priority: 11.05.1993 US 60230; 11.05.1993 US 60231
(43) Date of publication of application: 17.11.1994
(73) Proprietor: WISCONSIN ALUMNI RESEARCH FOUNDATION, Madison, WI 53705 (US)
(72) Inventor: Deluca, Hector Floyd, Deerfield, Wisconsin 53531 (US); Sicinski, Rafal R., 02-093 Warsaw, Pasteura 1 (PL)
(74) Representative: Ellis-Jones, Patrick George Armine

(56) References cited:
- EP-A- 0 205 285
- EP-A- 0 363 211
- EP-A- 0 528 209

## Description

This invention relates to biologically active vitamin D₃ compounds. More specifically, the invention relates to1α-hydroxylated-22-iodinated vitamin D₃ compounds and a process for the preparation thereof.

### Background and Summary of the Invention

The 1α-hydroxylated metabolites of vitamin D -- most importantly 1α,25-dihydroxyvitamin D₃ and 1α,25-dihydroxyvitamin D₂ -- are known as highly potent regulators of calcium homeostasis in animals and humans, and more recently their activity in cellular differentiation has also been established. V. Ostrem et al, Proc. Natl. Acad. Sci. USA, (1987), 84, 2610. As a consequence, many structural analogs of these metabolites, such as compounds with different side chain structures, different hydroxylation patterns, or different stereochemistry, have been prepared and tested. Important examples of such analogs are 1α-hydroxyvitamin D₃, 1α-hydroxyvitamin D₂, various side chain fluorinated derivatives of 1α,25-dihydroxyvitamin D₃, and side chain homologated analogs. Several of these known compounds exhibit highly potent activity in vivo or in vitro, and some of these have been found to exhibit an interesting separation of activities in cell differentiation and calcium regulation. This difference in activity provides these compounds with advantageous therapeutic activity profiles and thus numerous of these compounds are in use, or have been proposed for use, in the treatment of a variety of diseases such as renal osteodystrophy, vitamin D-resistant rickets, osteoporosis, psoriasis, and certain malignancies.

In a continuing effort to explore pharmacologically important vitamin D analogs, several new 22-iodinated vitamin D₃ compounds have now been synthesized. The 22-iodinated compounds show relatively high binding affinity for the vitamin D receptor thus demonstrating their potential for high in vivo biological activity. Further, these compounds induced relatively high differentiation of malignant cells. Also, these 22-iodo compounds showed high in vivo calcium transport activity with little or no bone calcium mobilization activity. Accordingly, the 22-iodo compounds show promise in the treatment of osteoporosis, particularly senile osteoporosis and postmenopausal osteoporosis. The iodo compounds can also be labeled with radio iodine to be used for 1α,25-(OH)₂-D₃ assays.

### Brief Description of the Drawings

Fig. 1 is a graph of the percent differentiation of HL-60 cells versus concentration for a prior art vitamin D₃ compound and two of the new 22-iodo-vitamin D₃ compounds; and
Fig. 2 is a graph of the competitive binding ability versus concentration for the same three compounds as in Fig. 1.

### Disclosure of the Invention

The present invention is directed to biologically active 1α-hydroxy-22-iodinated vitamin D₃ compounds and to a process for the preparation of biologically active 1α-hydroxy-22-iodinated vitamin D₃ compounds having the general formulas I and II shown below: 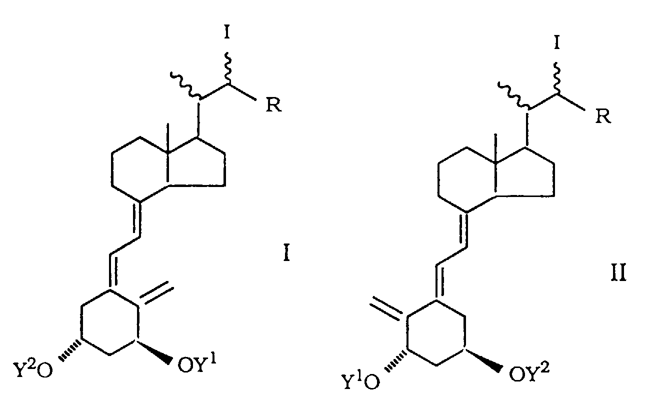 where Y¹ and Y², which may be the same or different, are each selected from the group consisting of hydrogen and a hydroxy-protecting group, and where R is hydrogen, an aryl, alkyl, hydroxyalkyl or fluoroalkyl group, or R may represent the following side chain fragment: 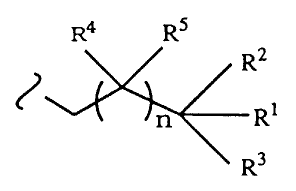 wherein R¹ represents hydrogen, hydroxy or protected hydroxy, R² and R³ are each selected from the group consisting of alkyl, hydroxyalkyl and fluoroalkyl, or, when taken together represent the group -- (CH₂)ₘ -- where m is an integer having a value of from 2 to 5, R⁴ is selected from the group consisting of hydrogen, hydroxy, fluorine, O-acyl, alkyl, hydroxyalkyl and fluoroalkyl, R⁵ is selected from the group consisting of hydrogen, fluorine, alkyl, hydroxyalkyl and fluoroalkyl, and wherein n is an integer having a value of from 1 to 5.

Specific important examples of side chains are the structures represented by formulas (a), (b), (c) and (d) below, i.e. the side chain as it occurs in (22S)-iodo-25-hydroxyvitamin D₃ (a); (22S)-iodovitamin D₃ (b); (22R)-iodo-25-hydroxyvitamin D₃ (c); and (22R)-iodovitamin D₃ (d).

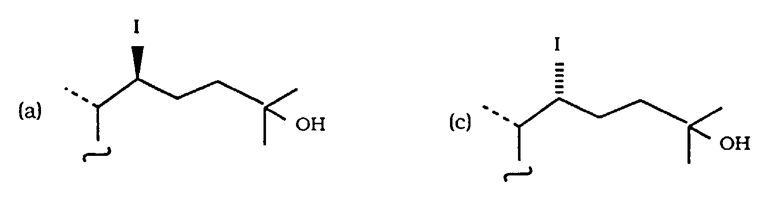

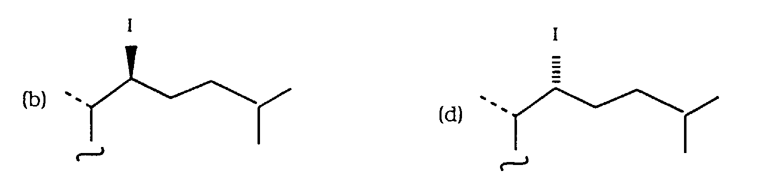

As used in the description, and in the claims, the term "hydroxy-protecting group" refers to any group commonly used for the protection of hydroxy functions during subsequent reactions, including, for example, acyl or alkylsilyl groups such as trimethylsilyl, triethylsilyl, t-butyldimethylsilyl and analogous alkyl or arylsilyl radicals, or alkoxyalkyl groups such as methoxymethyl, ethoxymethyl, methoxyethoxyethyl, tetrahydrofuranyl or tetrahydropyranyl. A "protected-hydroxy" is a hydroxy function derivatized by one of the above hydroxy-protecting groupings. "Alkyl" represents a straight-chain or branched hydrocarbon radical of 1 to 10 carbons in all its isomeric forms, such as methyl, ethyl, propyl, isopropyl, butyl, isobutyl, pentyl, etc., and the terms "hydroxyalkyl," "fluoroalkyl" and "arylalkyl" refer to such an alkyl radical substituted by one or more hydroxy, fluoro or aryl groups respectively. An "acyl" group is an alkanoyl group of 1 to 6 carbons in all its isomeric forms, or an aroyl group, such as benzoyl, or halo-, nitro- or alkyl-substituted benzoyl groups, or an alkoxycarbonyl group of the type alkyl-O-CO-, such as methoxycarbonyl, ethoxycarbonyl, propyloxycarbonyl, etc., or a dicarboxylic acyl group such as oxalyl, malonyl, succinoyl, glutaroyl, or adipoyl. The term "aryl" signifies a phenyl-, or an alkyl-, nitro- or halo-substituted phenyl group. The term alkoxy signifies the group alkyl-O-.

The wavy lines to the substituents at C-20 (methyl) and C-22 (iodine) indicate that these substituents may have either the R or S configuration.

The preparation of 1α-hydroxy-22-iodovitamin D compounds having the basic structures shown above can be accomplished starting from the diene-protected derivative of structure III or vitamin D compounds of the general structures IV and V 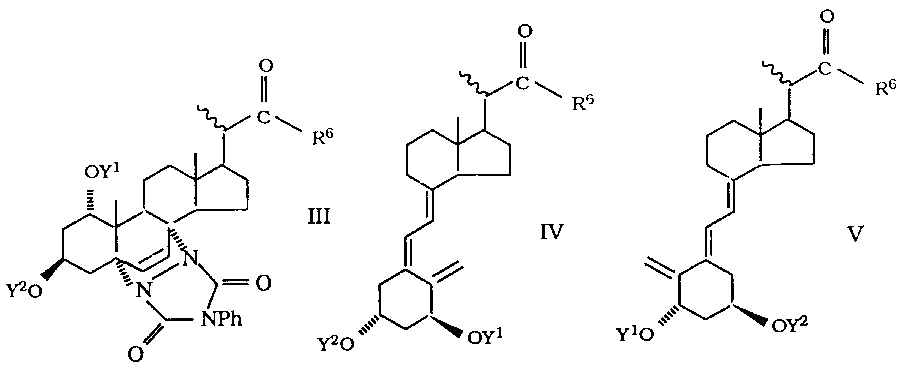 where Y¹ and Y2 are as defined above and R⁶ represents hydrogen, O-alkyl or O-aryl. Reaction of the C-22 aldehydes III, IV or V (R⁶ = hydrogen) with an organometallic reagent such as an alkylmagnesium halide having the structure RMgX (R as defined above, X = halogen) or alkyllithium reagent having the structure RLi (R as defined above), in the appropriate inert solvent, provides 22-hydroxy compounds of the general formulas VI, VII and VIII, respectively (Y³ = H). Alternatively, the same compounds VI-VIII can be obtained by reaction of the above mentioned organometallic reagents with esters III, IV and V (R⁶ = O-alkyl or O-aryl) under carefully controlled reaction conditions providing 22-ketones of the general formulas III, IV and V (R⁶ = R as defined above) and by subsequent reduction of the 22-oxo group in these compounds by the appropriate reducing agents (e.g. hydride reduction). 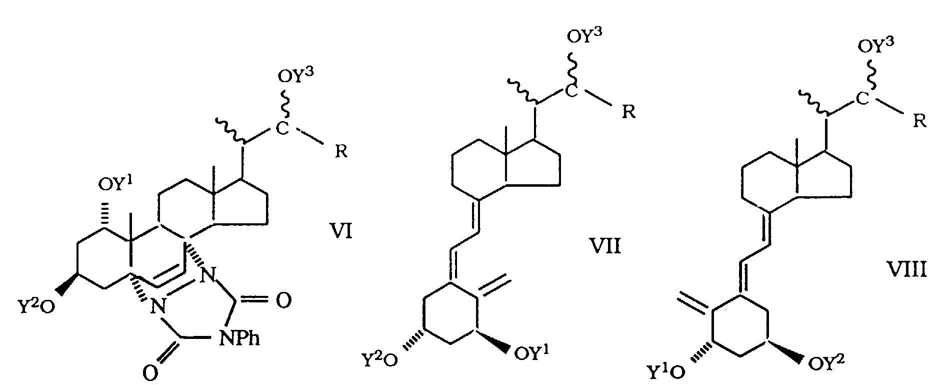 Adduct VI (Y³ = H) can then be converted to the intermediate VII or VIII, for example by being subjected to basic conditions to be converted by removal of the triazoline group to 5,7-diene steroid which is in turn converted via the well known process consisting of irradiation with UV light and thermal isomerization gives VII (Y³ = H), typically via solvent at a temperature from 50° to 90°C.

The 22-hydroxy intermediates VII and VIII (Y³ = H) having, preferably, all remaining hydroxy groups protected are then treated with an alkyl- or arylsulfonylhalide (e.g. methanesulfonylchloride, p-toluenesulfonylchloride) in a suitable solvent (e.g. pyridine) to obtain the corresponding 22-O-alkyl- or -arylsulfonyl derivatives (the compounds having the structures shown VII and VIII above, where Y³ is alkyl-SO₂- or aryl-SO₂-). These sulfonates are then subjected to nucleophilic substitution reaction with a reagent which can serve as an iodine ion source (inorganic iodides e.g. alkali metal iodides, ammonium iodide etc. and organic iodides e.g. tetraalkylammonium iodides etc.) in an appropriate solvent (preferably acetone, 2-butanone, 2-propanol, acetonitrile etc.), at a temperature ranging from 0°C to the boiling temperature of the solvent, thereby displacing the sulfonate group and obtaining the 22-iodo compounds, represented by the structures I and II above. To avoid undesired isomerization of the 5,6-double bond which can occur due to evolution of small amounts of iodine (radical dissociation of C-I bond) in the reaction medium it is required to perform such substitution reaction in the presence of mercury and in the absence of light of a wavelength close to the carbon-iodine bond absorption wavelength, i.e. about 259 nm, especially 254 to 264 nm, and preferably in the dark. In order to neutralize strong acids which can be formed in the side processes (concurrent elimination reactions) it is advantageous to add calcium carbonate to the reaction medium.

It is also evident that the reaction of the 22-sulfonates with isotopically labeled iodides (e.g. NH₄¹²³I, Na¹²⁵I, Na ¹²⁹I, Na¹³¹I, etc.) provides a convenient means for preparing 22-iodo compounds I and II in isotopically-labeled form. Due to known susceptibility of aliphatic iododerivatives to nucleophilic substitution such radioiodinated compounds can also be prepared by reaction of the unlabeled derivatives I or II with the isotopically labeled iodides (i.e. isotope exchange process). Vitamins of high specific activity (up to 2200 Ci/mmol) labeled with carrier-free radioiodine can be conveniently produced in that manner. These radiolabeled vitamin D derivatives find ready application as tracers in various known binding assays and for other experimental research studies.

The next step of the process comprises the removal of the hydroxy protecting groups to produce the free hydroxy compounds represented by 1α-hydroxyvitamin structures I and II above (where Y¹, Y² and R¹ are hydrogens) or 1α,25-dihydroxyvitamin D structures (where Y¹, Y² are hydrogens and R¹ is hydroxyl). Alternatively, the deprotection of hydroxyl groups may be performed on 22-sulfonates to obtain compounds of structures VII and VIII (where Y3 is alkyl-SO₂- or aryl-SO₂- and Y¹, Y2 and R¹ are hydrogens) which can be then subjected to substitution reaction giving rise to 22-iodo compounds I and II (where Y¹, Y² and R¹ are hydrogens). If obtained as a mixture of the two C22 epimers, 22-alcohols, sulfonates and/or iodides can be separated by chromatographic methods. If desired, the 5.6-cis compounds I, IV and VII can be easily converted to the corresponding 5,6-trans counterparts (and vice versa) II, V and VIII, respectively by the known iodide-catalyzed isomerization process.

This invention is more specifically described by the following illustrative examples. In these examples specific products identified by Arabic numerals (e.g. 1, 2, 3, etc.) refer to the specific structures so identified in the preceding description and in the Schemes.

### Example 1

### Reaction of vitamin D C-22 aldehyde 1 with Grignard reagent derived from bromocompound A (Scheme 1)

A solution of known 4-bromo-2-methyl-2(triethylsilyloxy) butane (A) (94 mg, 0.33 mmol) in anhydrous ether (0.3 mL) was added dropwise to a stirred mixture of magnesium powder (9.7 mg, 0.4 mmol; ∼50 mesh, Aldrich) in anhydrous ether (0.2 mL) under argon at room temperature with occasional warming it up to 35°C. After addition was complete the mixture was stirred for 15 min at room temperature and 30 min at 40°C. Then it was cooled to 0°C and a solution of known C-22 aldehyde (1) [32 mg, 0.056 mmol; see A. Kutner et al., J. Org. Chem. 53, 3450 (1988)] in anhydrous ether (0.3 mL, cooled to 0°C) was added dropwise. After the reaction mixture was stirred for 20 min at 0°C and 75 min at room temperature it was quenched with aqueous solution of NH₄Cl (2 mL) and diluted with 4:1 (v/v) benzene/ether (20 mL). The organic layer was separated, washed with water and diluted NaHCO₃, dried, and evaporated. TLC and HPLC control indicated formation of only one (22S) of the two possible isomers. Pure (22S)-hydroxyvitamin D derivative (2) was obtained as a colorless oil (31 mg, 72% yield) by preparative HPLC (Zorbax-Silica column 6.2 mm x 25 cm) using 3.5% ethyl acetate in hexane as an eluent; peak at 30 mL was collected. UV (EtOH) λₘₐₓ 264 nm, λₘᵢₙ 225 nm, A₂₆₄/A₂₂₅ = 1.6; ¹H-NMR (CDCl₃, 500 MHz): δ 0.062 (12 H, br s, 4 x SiMe), 0.546 (3 H, s, 18-H₃), 0.583 (6 H, q, J = 8 Hz, 3 x SiCH₂), 0.877 (18 H, s, 2 x Si-t-Bu), 0.920 (3 H, d, J = 6.4 Hz, 21-H₃), 0.947 (9 H, t, J = 8 Hz, 3 x SiCH₂CH₃), 1.221 and 1.225 (3 H and 3 H, each s, 26- and 27-H₃), 2.83 (1 H, br d, J = 12.5 Hz, 9β-H), 3.63 (1 H, m, 22-H), 4.19 (1 H, m, 3α-H), 4.37 (1 H, m, 1β-H), 4.87 and 5.18 (1 H and 1 H, each s, 19-H₂), 6.02 (1 H, d, J = 11.2 Hz, 7-H), 6.24 (1 H, d, J = 11.2 Hz, 6-H); MS, m/z (rel intensity) 774 (M⁺, 15), 642 (43), 75 (100); exact mass calcd for C₄₅H₈₆O₄Si₃ 774.5834, found 774.5850.

### Example 2

### Reaction of 22-hydroxyvitamin D compound (2) with p-toluenesulfonyl chloride

To a solution of alcohol (2) (28 mg, 0.036 mmol) in dry pyridide (100 µL) was added freshly recrystallized p-toluenesulfonyl chloride and the reaction was allowed to proceed for 64 h at 4°C. The reaction mixture was poured into ice/saturated NaHCO₃ with stirring. After 40 min of stirring the aqueous suspension was extracted with 4:1 (v/v) benzene/ether (3 x 10 mL). The combined organic extracts were washed with saturated NaHCO₃, water, saturated CUSO₄, again water, dried (Na₂S0₄) and evaporated. The oily yellowish residue was purified by preparative HPLC (Zorbax-Silica column 6.2 mm x 25 cm) using 2% ethyl acetate in hexane as an eluent. Pure tosylate (3) (26 mg, 78%; collected at 20 mL) was obtained as a colorless oil: UV (hexane) λₘₐₓ 264 and 223 nm, λₘᵢₙ 238 nm; ¹H-NMR (CDCl₃, 500 MHz) : δ 0.059 and 0.067 (6 H and 6 H, each s, 2 x SiMe₂), 0.479 (3 H, s, 18-H₃), 0.528 (6 H,q, J = 8 Hz, 3 x SiCH₂), 0.877 (18 H, s, 2 x Si-t-Bu), 0.915 (9 H, t, J = 8 Hz, 3 x SiCH₂CH₃), 0.929 (3 H, d, J = 6.0 Hz, 21-H₃), 1.103 and 1.141 (3 H and 3 H, each s, 26- and 27- H₃), 2.43 (3 H, s, Ar-Me), 2.80 (1 H, br d, J = 12.3 Hz, 9β-H), 4.19 (1 H, m, 3α-H), 4.38 (1 H, m, 1β-H), 4.58 (1 H, t, J = 7.1 Hz, 22-H), 4.86 and 5.19 (1 H and 1 H, each s, 19-H₂), 5.99 (1 H, d, J = 11.2 Hz, 7-H), 6.22 (1 H, d, J = 11.2 Hz, 6-H), 7.32 (2 H, d, J = 8 Hz, Ar-H), 7.80 (2 H, d, J = 8 Hz, Ar-H); MS, m/z (rel intensity) 928 (M⁺, 1), 796 (2), 756 (3), 664 (3), 624 (47), 492 (27), 173 (100); exact mass calcd for C₅₂H₉₂O₆Si₃S 928.5922, found 928.5894.

### Example 3

### Reaction of vitamin D 22-p-toluenesulfonate (3) with sodium iodide

To a stirred solution of tosylate (3) (4.6 mg, 5 µmol) in 1:1 (v/v) acetone/2-butanone (200 µL) was added a drop of mercury (220 mg) and anhydrous calcium carbonate (1 mg, 10 µmol) followed by sodium iodide (3.7 mg, 25 µmol). The resultant mixture was stirred and heated for 100 h at 45°C in the dark under argon, by which time almost no starting material remained. The reaction mixture was poured into water (10 mL) and extracted with ethyl acetate (2 x 10 mL). The combined organic layers were washed with 1% Na₂S₂0₃ and water, dried (Na₂SO₄) and evaporated. The work up of the reaction mixture and following chromatographic separations were done using subdued light in the laboratory and exposure of products to any light, particularly of wavelength close to the carbon-iodine bond absorption (259 nm) was avoided as much as possible. The mixture of products was then repeatedly chromatographed by HPLC (6.2 mm x 25 cm Zorbax-Silica column) using 0.1% ethyl acetate in hexane as an eluent. The peaks of 22S-iodocompound (4) and 22R-isomer (5) partially overlapped (Rᵥ's 48 mL and 53 mL, respectively) but rechromatography (or recycling of both peaks) afforded pure material.
(22S)-iodocompound (4) (0.8 mg, 18%): UV (hexane) λₘₐₓ 264 nm, λₘᵢₙ 227 nm; A₂₆₄/A₂₂₇ ) 1.6; ¹H-NMR (CDCL₃, 500 MHz) δ 0.058 and 0.064 (6 H and 6 H, each s, 2 x SiMe₂), 0.569 (6 H, q, J = 8 Hz, 3 x SiCH₂), 0.597 (3 H, s, 18-H₃), 0.875 (18 H, br s, 2 x Si-t-Bu), 0.944 (9 H, t, J = 8 Hz, 3 x SiCH₂CH₃), 0.964 (3 H, d, J = 6 Hz, 21-H₃), 1.194 and 1.211 (3H and 3H, each s, 26-and 27-H₃), 2.82 (1 H, br d, J ∼ 13 Hz, 9β-H), 4.19 (2 H, m, 3α-and 22-H), 4.37 (1 H, m, 1β-H), 4.86 and 5.18 (1 H and 1 H, each s, 19-H₂), 6.02 (1 H, d, J = 11.2 Hz, 7-H), 6.23 (1 H, d, J = 11.2 Hz, 6-H); MS, m/z (rel intensity) 884 (M⁺, 21), 752 (62), 624 (22), 248 (100); exact mass calcd for C₄₅H₈₅O₃Si₃I 884.4851, found 884.4875.
(22R)-iodocompound (5) (0.4 mg, 9%): UV (hexane) λmax 264 nm, λₘᵢₙ 227 nm; A₂₆₄/A₂₂₇ = 1.8; ¹H-NMR δ 0.064 (12 H, br s, 4 x SiMe), 0.517 (3 H, s, 18-H₃), 0.568 (6 H, q, J = 8 Hz, 3 x SiCH₂), 0.875 (18 H, br s, 2 x Si-t-Bu), 0.950 (9 H, t, J = 8 Hz, 3 x SiCH₂CH₃), 1.158 (3H, d, J = 6.8 Hz, 21-H₃), 1.179 (6 H, br s, 26- and 27-H₃), 2.83 (1 H, br d, J ∼ 13 Hz, 9β-H), 4.19 (1 H, m, 3α-H), 4.37 (1 H, m, 1β-H), 4.45 (1 H, d, J = 11 Hz, 22-H), 4.86 and 5.18 (1 H and 1 H, each s, 19-H₂), 6.02 (1 H, d, J = 11.2 Hz, 7-H), 6.23 (1 H, d, J = 11.2 Hz, 6-H); MS, m/z (rel intensity) 884 (M⁺, 20), 752 (54), 624 (24), 248 (100); exact mass calcd for C₄₅H₈₅O₃Si₃I 884.9851, found 884.4899. Both iodovitamins (4) and (5), when protected from light can be stored for a prolonged time in a freezer with neither 5,6-cis/trans isomerization nor epimerization at C-22. If desired, however, both processes can be easily performed. In order to achieve the interconversion between both C(22)-epimers each compound (4) and (5) can be subjected to an analogous nucleophilic substitution reaction with NaI as described above for the tosylate (3). In both cases the similar equilibrium mixture can be obtained consisting of epimeric (22S)- and (22R)-iodocompounds (4) and (5) in a ratio of about 3-4:1. Transformation of 5,6-cis isomers to their 5,6-trans analogs is described in the following Example.

### Example 4

### 5,6-Double bond isomerization of 22-iodovitamin D compounds (4) and (5)

The synthetic process described in the Example 3 above ensures the preparation of pure 5,6-cis isomers (4) and (5) uncontaminated by 5,6-trans counterparts. Such geometrical isomers are easily distinguishable by both chromatographical and spectral properties. If required, iodine-catalyzed isomerization of 5,6-cis compounds (4) and (5) to the respective 5,6-trans products (6) and (7) can be accomplished according to a known procedure [see A. Verloop et al., Rec. Trav. Chim. Pays-Bas 78, 1004 (1959)]. Thus, treatment of compound (4) in ether with a catalytic amount of iodine [2% of the amount of (4)], while keeping the solution under diffuse daylight for 1h, results in cis to trans isomerization and after HPLC separation (Zorbax-Silica column 6.2 mm x 25 cm, 0.1% ethyl acetate in hexane), the 5,6-trans isomer (6) is obtained (Rᵥ 60 mL). Similarly, compound (5) upon treatment with iodine under the above conditions, is isomerized to (7), which can be obtained in pure form after HPLC (conditions as above) separation (Rᵥ 70 mL).
(6), ¹H-NMR (CDCl₃, 500 MHz) δ 0.609 (3 H, s, 18-H₃), 0.967 (3 H, d, J = 6 Hz, 21-H₃), 1.201 and 1.216 (3 H and 3 H, each s, 26- and 27-H₃), 2.88 (1 H, br d, J - 13 Hz, 9β-H), ca 4.2 (2 H, m, 3α- and 22-H), 4.57 (1 H, m, 1β-H), 4.95 and 4.99 (1 H and 1 H, each s, 19-H₂), 5.83 and 6.47 (1 H and 1 H, each d, J = 11 Hz, 7- and 6-H).
(7), ¹H-NMR (CDCl₃, 500 MHz) δ 0.532 (3 H, s, 18-H₃), 1.167 (3 H, d, J = 6 Hz, 21-H₃), 1.185 (6 H, br s, 26- and 27-H₃), 2.88 (1 H, br d, J - 13 Hz, 9β-H), 4.22 (1 H, m, 3α-H), 4.46 (1 H, d, J = 11 Hz, 22-H), 4.54 (1 H, m, 1β-H), 4.95 and 4.99 (1 H and 1 H, each s, 19-H₂), 5.83 and 6.46 (1 H and 1H, each d, J = 11 Hz, 7- and 6-H).

### Example 5

### Deprotection of hydroxyl groups in 22-iodovitamin D compounds (4) and (5)

Each of 22-iodovitamins was separately hydrolyzed using the same procedure. To a solution of protected triol (4) (1 mg) in anhydrous benzene (50 µL) was added AG 50W-X4 ion exchange resin (20 mg, prewashed with methanol) as a slurry in an anhydrous methanol (200 µL). A drop of mercury (400 mg) was added and the resultant mixture was vigorously stirred at room temperature for 10 h in the dark under argon. The reaction was diluted with 1:1 (v/v) ether/ethyl acetate (1 mL), the solution was decanted and transferred to a separatory funnel and the resin was washed with 1:1 ether/ethyl acetate (2 x 2 mL). The combined organic phase was washed with 5 mL portions of brine, 1% Na₂S₂0₃, saturated NaHC0₃, and brine again, dried (Na₂SO₄) and evaporated (temp. below 35°C). The work up of the reaction mixture and following chromatographic separations were done using subdued light in the laboratory and exposure of iodinated compounds to any light, particularly of wavelength close to the carbon-iodine absorption (259 nm), was avoided as much as possible. Preparative HPLC [6.2 mm x 25 cm Zorbax-Silica column, 1:1 (v/v) hexane/ethyl acetate as an eluent] provided small amount of (22R,25)-epoxy-1α-hydroxyvitamin D3 (8) (eluted at Rᵥ 34 mL). Further elution gave (22S)-iodo-1α,25-dihydroxyvitamin D₃ (9): Rᵥ 79 mL; UV (EtOH) λₘₐₓ 264 nm, λₘᵢₙ 227 nm, A₂₆₄/A₂₂₇ = 2.0; ¹H-NMR (CDC13, 500 MHz) δ 0.607 (3 H, s, 18-H₃), 0.975 (3 H, d, J = 5.9 Hz, 21-H₃), 1.23 and 1.24 (3 H and 3 H, each s, 26- and 27-H₃), 2.83 (1 H, br d, J ∼ 13 Hz, 9β-H), 4.21 (2 H, m, 3α- and 22-H), 4.44 (1 H, m, 1β-H), 5.00 and 5.33 (1 H and 1 H, each s, 19-H₂), 6.02 (1 H, d, J = 11.3 Hz, 7-H), 6.38 (1 H, d, J = 11.3 Hz, 6-H); MS, m/z (rel intensity) 542 (M⁺, 2), 524 (M⁺ - H₂0, 31), 506 (M⁺ - 2H₂0, 17), 414 (M⁺ - HI, 8), 396 (M⁺ - HI - H₂O, 54), 378 (M⁺ - HI - 2 H₂O, 21), 99 (100); exact mass calcd for C₂₇H₄₁O₂I (M⁺ - H₂O) 524.2151, found 524.2145.

Analogously performed hydrolysis of 22-iodocompound (5) gave after HPLC separation (conditions as above) a trace of (22S,25)-epoxy-1α-hydroxyvitamin D₃ (8) (eluted at Rᵥ 28 mL) and (22R)-iodo-1α,25-dihydroxyvitamin D₃ (11): Rᵥ 63 mL; UV (EtOH) λₘₐₓ 264 nm, λₘᵢₙ 228 nm, A₂₆₄/A₂₂₈ = 1.7; ¹H-NMR (CDCl₃, 500 MHz) δ 0.535 (3 H, s, 18-H₃), 1.172 (3 H, d, J = 6.5 Hz, 21-H₃), 1.224 (6 H, br s, 26- and 27-H₃), 2.82 (1 H, br d, J ∼ 13 Hz, 9β-H), 4.24 (1 H, m, 3α-H), 4.45 (1 H, m, 1β-H), 4.47 (1 H, d, J = 12 Hz, 22-H), 5.00 and 5.33 (1 H and 1 H, each s, 19-H₂), 6.02 (1 H, d, J = 11.2 Hz, 7-H), 6.37 (1 H, d, J = 11.2 Hz, 6-H); MS m/z (rel intensity) 542 (M⁺, 8), 524 (M⁺ - H₂0, 64), 506 (M⁺ - 2 H₂0, 19), 414(M⁺ - HI, 10), 396 (M⁺ - HI - H₂0, 62), 378 (M⁺ - HI - 2 H₂0, 9), 99 (100); exact mass calcd for C₂₇H₄₃0₃I 542.2257, found 542.2248; calcd for C₂₇H₄₁0₂I (M⁺-H₂0) 524.2151, found 524.2136.
Elongation of the hydrolysis time results in the formation of increasing proportions of cyclic ethers (8) or (10). These conversions are useful for correlating the respective C(22)-epimeric iodocompounds with starting tosylates. Thus, for example, (22S)-tosylate (3) subjected to the analogous (mercury is not necessary) hydrolysis conditions as described above formed (22R,25)-epoxy-1α-OH-D₃ 8 (63% yield) as the only isolable product.

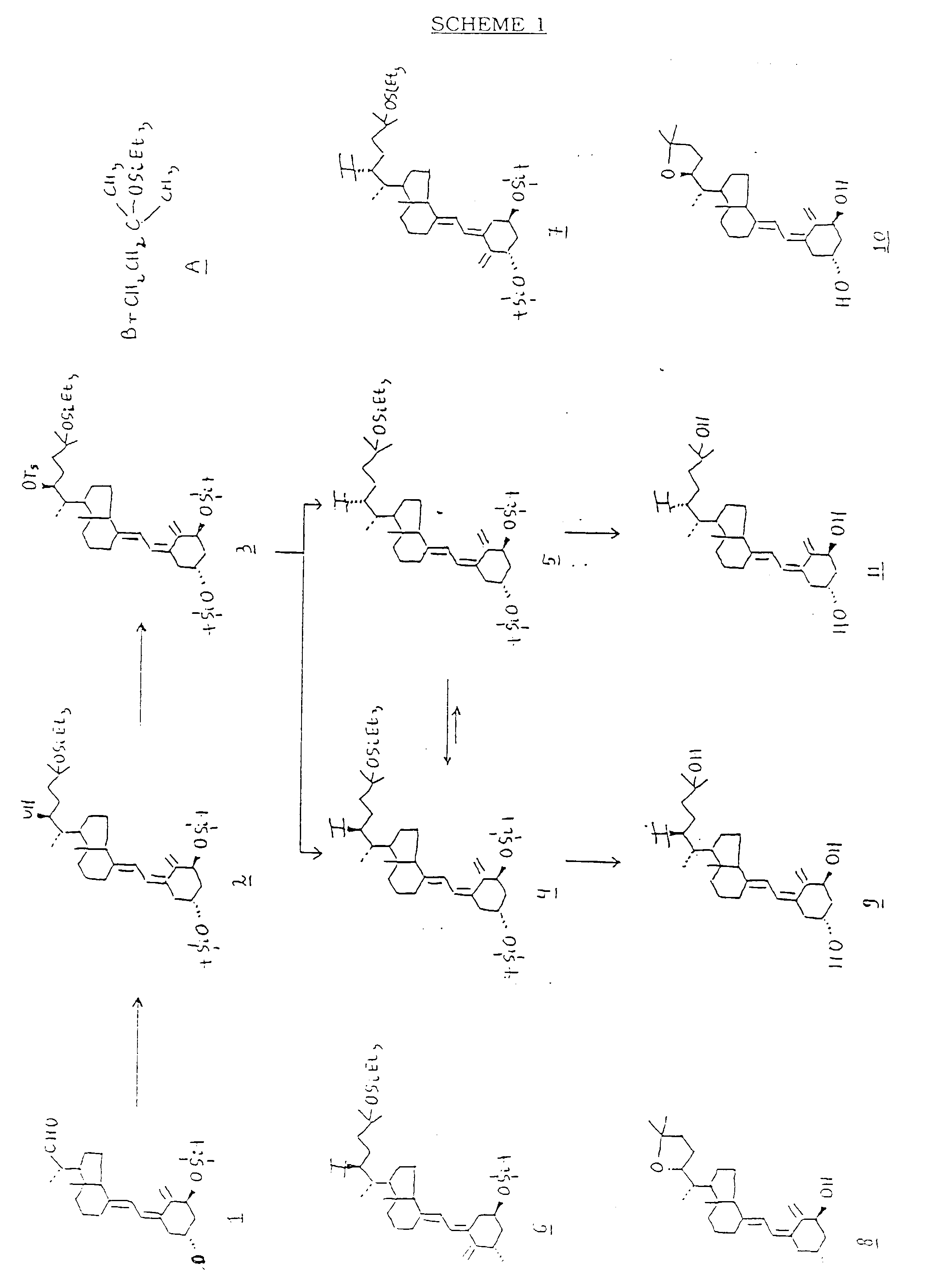

### Biological Activity of 1α-Hydroxy-22-Iodo-Vitamin D Compounds

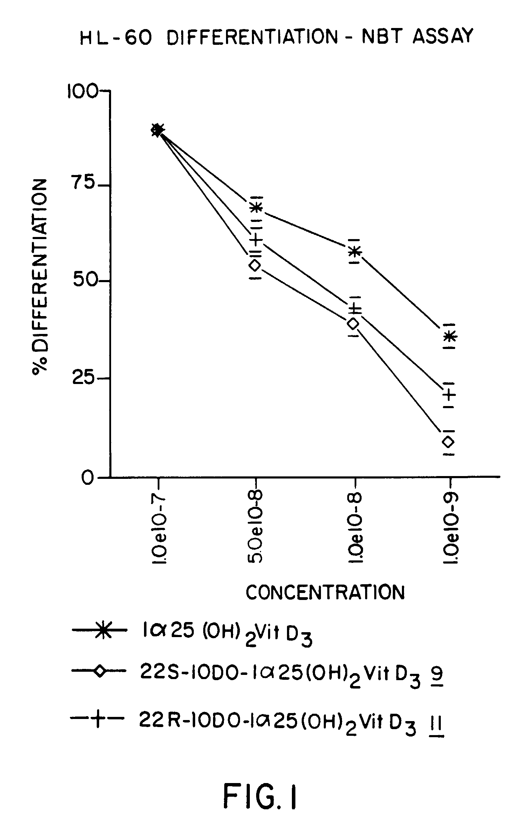

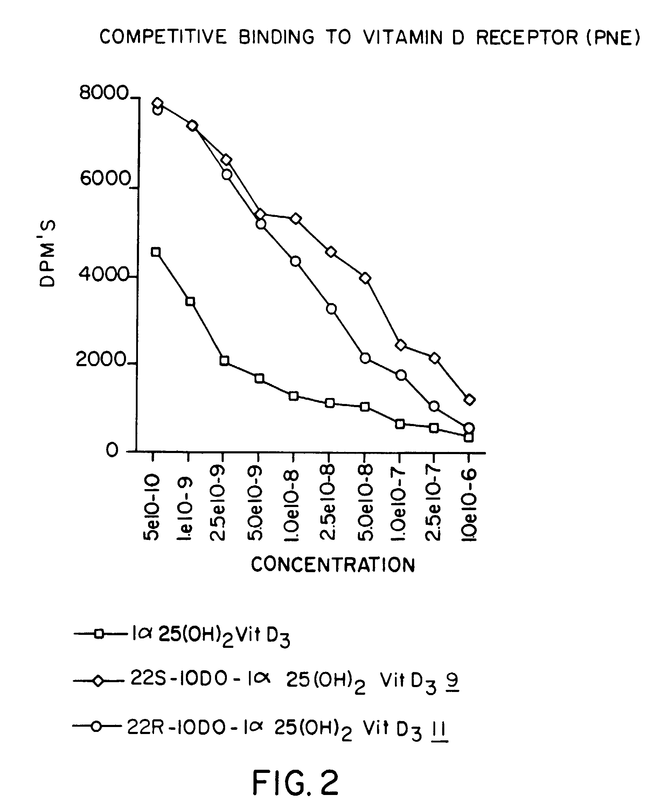

The novel compounds of this invention exhibit an unexpected pattern of biological activity. All of the 22-iodo compounds exhibited high potency in promoting the differentiation of malignant cells. Competitive binding tests also showed that these compounds have the potential for high in vivo biological activity. In addition, the 22-iodo compounds showed high in vivo calcium transport activity with little or no bone calcium mobilization activity. This is illustrated by the biological assay results obtained for the claimed 22-iodo-vitamin D₃ compounds, which are summarized in Figs. 1 and 2, and Table 1. Fig. 1 shows a comparison of the activity of the known active metabolite 1α,25-dihydroxyvitamin D₃ and two of the 22-iodo analogs in inducing the differentiation of human leukemia cells (HL-60 cells) in culture to normal cells (monocytes). Differentiation activity was assessed by a standard differentiation assay, abbreviated in Fig. 1 as NBT (nitroblue tetrazolium reduction). The assay was conducted according to known procedures, as given, for example, by DeLuca et al U. S. Patent 4,717,721 and Ostrem et al, J. Biol. Chem. 262, 14164, 1987. For the assay, the differentiation activity of the test compounds is expressed in terms of the percent of HL-60 cells having differentiated to monocytes in response to a given concentration of test compound.

The results summarized in Fig. 1 clearly show that the new 22-iodo-vitamin D₃ analogs and specifically (22S)-iodo-1α,25-dihydroxyvitamin D₃ and (22R)-iodo-1α,25-dihydroxyvitamin D₃, are as potent as 1α,25-dihydroxyvitamin D₃ in promoting the differentiation of leukemia cells. Thus in NBT assay close to 90% of the cells are induced to differentiation by 1α,25-dihydroxyvitamin D₃ at a concentration of 1 x 10⁻⁷ molar, and the same degree of differentiation is achieved by the two 22-iodo analogs.

Fig. 2 shows a comparison of the same three compounds as in Fig. 1 illustrating their relative activity with regard to competitive binding to the vitamin D receptor. The competitive receptor binding was done with pig nuclear extract (PNE) as described in Perlman et al. Biochemistry 29, 190-196 (1990) using the porcine extract prepared as described by Dame et al PNAS 82, 7825-7829 (1985). These data are used to demonstrate that the 22-iodo compounds, and in particular the 22S (compound 9) and 22R (compound 11) analogs tested, have potential for high in vivo biological activity.

In regard to the biological data on the calcemic activity of these compounds reported in Table 1, the data in Table 1 illustrate that (22S)-iodo-1α,25-dihydroxyvitamin D₃ has biological activity in intestinal calcium transport much higher, i.e. about double, to that of 1,25-(OH)₂D₃. The data also illustrates that this compound possesses little or no bone calcium mobilizing activity even when given at 1µg per day for six days.

The 22-iodo compounds would thus find utility as a treatment for osteoporosis, particularly postmenopausal osteoporosis and senile osteoporosis. The utility for these compounds results from their low bone calcium mobilizing activity with normal differentiative activity, normal binding to the receptor, and high calcium transport activity.

**TABLE 1**

| INTESTINAL CALCIUM TRANSPORT AND BONE CALCIUM MOBILIZATION ACTIVITY OF 22S-IODO-1,25-(OH)₂D₃ | | | |
|---|---|---|---|
| Compound | Dose (µg/d/6 days) | Intestinal Calcium Transport (Serosal/Mucosal Ratio) | Bone Calcium Mobilization (Serum Calcium) (mg/100 ml) |
| None (-D) | 0 | 4.0 ± 0.2 | 4.1 ± 0.1 |
| 1,25-(OH)₂D₃ | 0.1 | 11.2 ± 1.2 | 5.9 ± 0.3 |
| 22S-Iodo-1,25-(OH)₂D₃ | 1.0 | 22.8 ± 1.4 | 4.9 ± 0.1 |

Rats were fed the vitamin D-deficient, low calcium (0.02%) diet for 3 weeks and then given the indicated dose in 0.1 ml propylene glycol:ethanol (95:5) intraperitoneally each day for 6 days. On day 7, serum was analyzed for calcium and the duodenum used to measure intestinal calcium transport as described by Martin et al. Am. J. Physiol. 216, 1351-1359, 1969, as modified by Perlman et al. Biochemistry, 29, 190-196, 1990.

For treatment purposes, the novel compounds of this invention can be formulated as solutions in innocuous solvents, or as emulsions, suspensions or dispersions in suitable innocuous solvents or carriers, or as pills, tablets or capsules, containing solid carriers according to conventional methods known in the art. For topical applications the compounds are advantageously formulated as creams or ointments or similar vehicle suitable for topical applications. Any such formulations may also contain other pharmaceutically-acceptable and non-toxic excipients such as stabilizers, anti-oxidants, binders, coloring agents or emulsifying or taste-modifying agents.

The compounds are advantageously administered by injection, or by intravenous infusion of suitable sterile solutions, or in the form of oral doses via the alimentary canal, or topically in the form of ointments, lotions, or in suitable transdermal patches. For the treatment of malignant diseases, the 22-iodo-vitamin D compounds of this invention are administered to subjects in dosages sufficient to inhibit the proliferation of malignant cells and induce their differentiation into normal monocyte-macrophages. Suitable dosage amounts are from 0.5 to 500 µg of compound per day, such dosages being adjusted, depending on the disease to be treated, its severity and the response or condition of the subject as is well-understood in the art.

## Claims

1. A vitamin D compound having the structure I or II shown below: 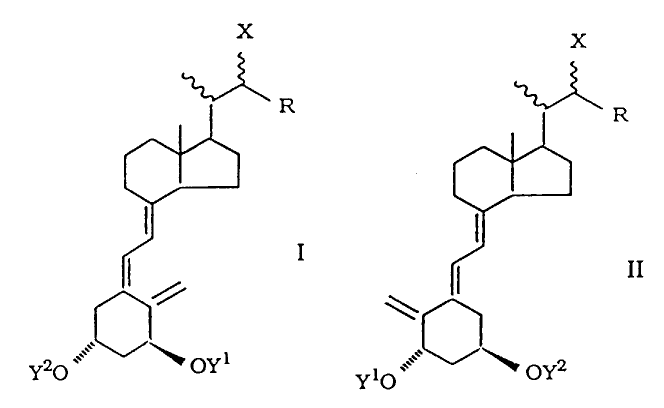 where X is iodine or radiodine, Y¹ and Y², which may be the same or different, are hydrogen or a hydroxy-protecting group, and R is hydrogen or an aryl, alkyl, hydroxyalkyl or fluoroalkyl group, or the following side chain fragment: 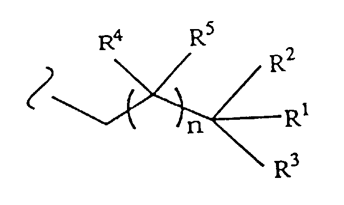 wherein R¹ represents hydrogen, hydroxy or protected hydroxy, R² and R³, which may be the same or different, are an alkyl, hydroxyalkyl or fluoroalkyl group or, when taken together, represent the group --[CH₂] -- where m is an integer from 2 to 5, R⁴ is hydrogen, hydroxy or fluorine or an O-acyl, alkyl, hydroxyalkyl or fluoroalkyl group, R⁵ is hydrogen or fluorine or an alkyl, hydroxyalkyl or fluoroalkyl group and n is an integer from 1 to 5.

2. 22S- or 22R-iodo-vitamin D₃.

3. 22S- or 22R-iodo-25-hydroxyvitamin D₃.

4. 22S -or 22R-iodo-1α-hydroxyvitamin D₃.

5. 22S -or 22R-iodo-1α-25-dihydroxyvitamin D₃.

6. A vitamin D compound according to claim 1 wherein said radioiodine is ¹²³I, ¹²⁵I, ¹²⁹I, or ¹³¹I.

7. A method for preparing a 22-iodo-vitamin D₃ compound comprising reacting a 22-hydroxy-intermediate of formula VII or VIII: 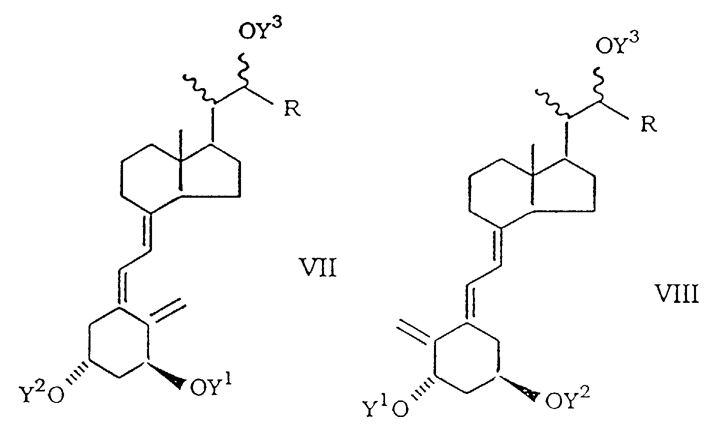 where Y¹ and Y², which may be the same or different, are hydroxy-protecting groups, Y³ is hydrogen, R is as defined in claim 1 with an alkylsulfonylhalide or an arylsulfonylhalide in a solvent to obtain the corresponding 22-O-alkyl- or 22-O-aryl-sulfonyl derivative, reacting the sulfonyl derivative with an iodide ion source in a solvent at a temperature from 0°C to the boiling temperature of the solvent in the presence of mercury and in the absence of light of a wavelength close to the carbon-iodine bond absorption wavelength, to obtain an 22-iodo compound of formula I and II where Y¹ and Y² are hydroxy-protecting groups and, optionally, either subsequently removing the hydroxy-protecting groups Y¹ and Y², or removing said groups after obtaining the sulfonyl derivative and before reaction with the iodide ion source, to provide compounds of formula I or II where Y¹ and Y² are both hydrogen.

8. A method according to claim 7 wherein 22-hydroxy-intermediate is prepared by reacting an aldehyde of formula IV or V: 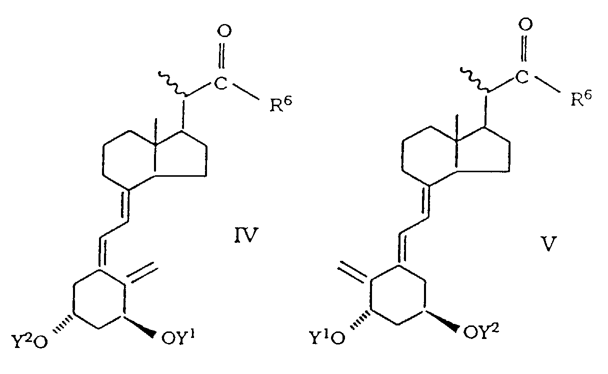 where Y¹ arid Y² are hydroxy-protecting groups and R⁶ represents hydrogen, with an organometallic reagent in an inert solvent.

9. A method according to claim 7 wherein the 22-hydroxy intermediate is prepared by reacting a compound of formula IV or V 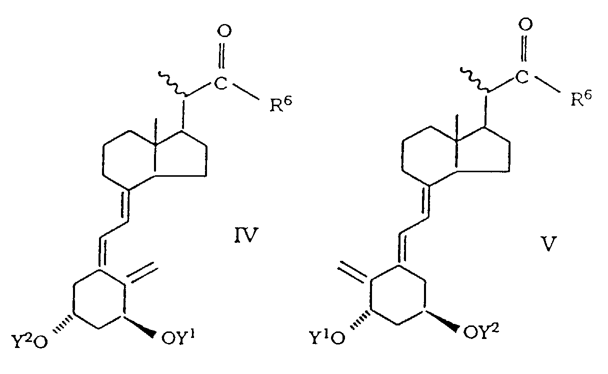 where Y¹ and Y², which may be the same or different, are hydrogen or a hydroxy-protecting group, and R₆ represents O-alkyl or O-aryl, with an organometallic reagent to provide a 22-ketone compound of the formula IV or V where R⁶ represents R as defined in claim 7, protecting any hydroxy groups if required; and reducing the 22-oxo group of said 22-ketone compound with an appropriate reducing agent.

10. A method according to claim 7 wherein the 22-hydroxy intermediate is prepared by reacting a diene-protected aldehyde derivative of formula III: 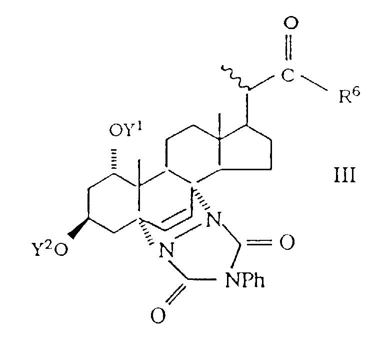 where Y¹ and Y² are hydroxy-protecting groups and R⁶ represents hydrogen, with an organometallic reagent in an inert solvent, to produce an adduct of formula VI 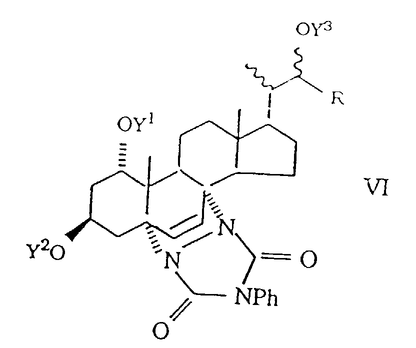 where R is as defined in claim 7 and Y³ is hydrogen, and converting said adduct VI to the desired 22-hydroxy-intermediate by by removal of the triazoline group to form a 5,7-diene steroid intermediate, subjecting said 5,7-diene steroid intermediate to irradiation with ultraviolet light to obtain the corresponding previtamin D compound, and isomerizing said previtamin D compound in a solvent at a temperature from 50°C to 90°C.

11. A method according to claim 7 wherein the 22-hydroxy intermediate is prepared by reacting a diene-protected ester derivative of formula III 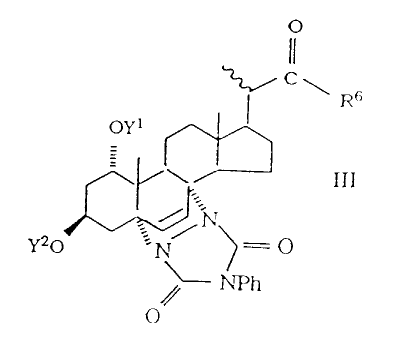 where Y¹ and Y² are as defined in claim 9 and R⁶ represents O-alkyl or O-aryl, with an organometallic reagent, to produce a 22-ketone compound of formula III where R⁶ represents R as defined in claim 7, protecting any hydroxy groups if required; and reducing the 22-oxo group of said 22-ketone compound with an appropriate reducing agent to produce an adduct of formula VI as defined in claim 10 and converting said adduct VI to the desired 22-hydroxy-intermediate by removal of the triazoline group to form a 5,7-diene steroid intermediate, subjecting said 5,7-diene steroid intermediate to irradiation with ultraviolet light to obtain the corresponding previtamin D compound, and isomerizing said previtamin D compound in a solvent at a temperature from 50°C to 90°C.

12. A method according to any one of claims 7 to 11 wherein the organometallic reagent is an alkylmagnesium halide having the structure RMgX or an alkyllithium having the structure RLi where R is as defined in claim 7 and X is a halogen.

13. A method according to any one of claims 7 to 12 wherein the iodide is an tetraalkylammonium iodide, alkali metal iodide or ammonium iodide or a mixture thereof.

14. A method according to any one of claims 7 to 13 wherein the step of reacting the sulfonyl derivative with the iodide ion source occurs in the absence of light having a wavelength of about 259 nm.

15. A method according to any one of claims 7 to 14 which also includes adding mercury to the reaction medium during any one of the reaction steps involving iodide compounds.

16. A method according to any one of claims 7 to 15 which also includes adding calcium carbonate to the reaction medium during nucleophilic substitution of the sulfonyloxy derivative by iodide ion.

17. A pharmaceutical composition comprising a 22-iodo-vitamin D compound as claimed in any one ot claims 1 to 6 or one obtained by a method as claimed in any one of the claims 7 to 16 together with a pharmaceutically acceptable excipient.

18. A composition according to claim 17 wherein the 22-iodo-compound is in solution in a liquid ingestible and non toxic vehicle and the solution is encapsulated.

19. A composition according to claim 17 which is a slow release formulation.

20. A 22-iodo-vitamin D₃ compound as defined in claim 1 for use in the treatment of osteoporosis.

21. Use according to claim 20 wherein the 22-iodo-vitamin D₃ compound is administered in an amount of from 0.5 µg to 500 µg per day.

22. Use according to claims 20 or 21 for senile osteoporosis.

23. Use according to claims 20 or 21 for postmenopausal osteoporosis.

24. Use according to any one of claims 20 to 23 wherein the compound is administered to women during and subsequent to menopause.

25. Use according to any one of claims 20 to 23 wherein the compound is administered to women prior to the onset of menopause.

26. Use according to any one of claims 20 to 25 for low bone turnover osteoporosis.

27. Use according to any one of claims 20 to 26 wherein the compound is administered daily in divided dosages.

## Patentansprüche

1. Vitamin D-Verbindung mit der unten dargestellten Struktur I oder II: 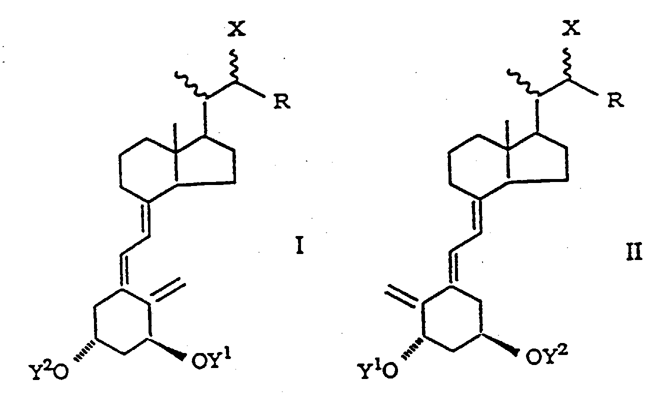 worin X Jod oder Radiojod ist, Y¹ und Y², die dieselben oder verschieden sein können, sind Wasserstoff oder eine Hydroxylschutzqruppe und R ist Wasserstoff oder eine Aryl-, Alkyl-, Hydroxyalkyl- oder Fluoralkylgruppe oder das folgende Seitenkettenfragment: 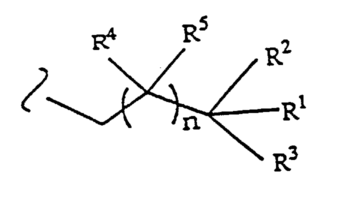 worin R¹ Wasserstoff wiedergibt, Hydroxy oder geschütztes Hydroxy, R² und R³, die dieselben oder verschieden sein können, eine Alkyl-, Hydroxyalkyl- oder Fluoralkylgruppe oder, wenn zusammen genommen, die Gruppe -[CH₂]ₘ- wiedergeben, wobei m eine ganze Zahl von 2 bis 5 ist, R⁴ Wasserstoff Hydroxy oder Fluor oder eine O-Acyl-, Alkyl-, Hydroxyalkyl- oder Fluoralkylgruppe ist, R⁵ Wasserstoff oder Fluor oder eine Alkyl-, Hydroxyalkyl- oder Fluoralkylgruppe und n eine ganze Zahl von 1 bis 5.

2. 22S- oder 22R-Jodo-Vitamin D₃.

3. 22S- oder 22R-Jodo-25-Hydroxyvitamin D₃.

4. 22S- oder 22R-Jodo-la-Hydroxyvitamin D₃.

5. 22S- oder 22R-Jodo-1α-Dihydroxyvitamin D₃.

6. Vitamin D-Verbindung gemäß Anspruch 1, bei der das Radiojod ¹²³I, ¹²⁵I, ¹²⁹I oder ¹³¹I ist.

7. Verfahren zur Herstellung einer 22-Jod-Vitamin D₃-Verbindung, umfassend die Umsetzung einer 22-Hydroxyzwischenstufe der Formel VII oder VIII: 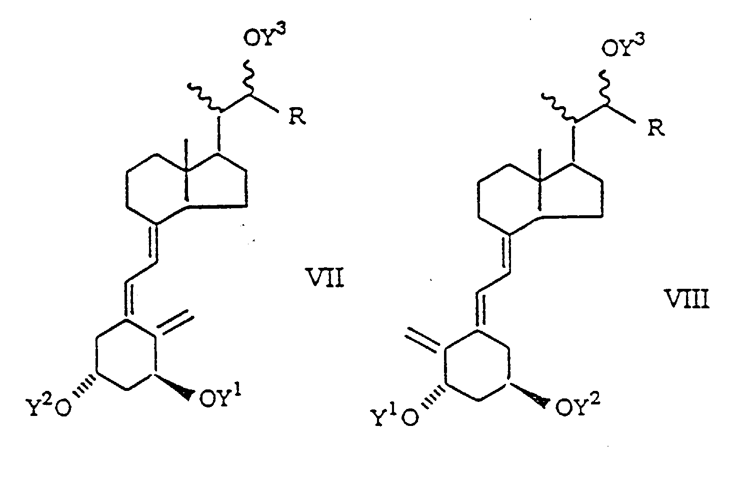 worin Y¹ und Y², die dieselben oder verschieden sein können, Hydroxylschutzgruppen sind, Y³ Wasserstoff ist und R wie in Anspruch 1 definiert ist,
mit einem Alkylsufonylhalogenid oder einem Arylsulfonylhalogenid in einem Lösungsmittel, um das entsprechende 22-O-Alkyl- oder 22-O-Arylsulfonylderivat zu erhalten, Umsetzen des Sulfonylderivats mit einer Jodidionenquelle in einem Lösungsmittel bei einer Temperatur von 0°C bis zum Siedepunkt des Lösungsmittels in Gegenwart von Quecksilber und in Abwesenheit von Licht einer Wellenlänge nahe der Absorptionswellenlänge der Kohlenstoff-Jod-Bindung, um eine 22-Jodoverbindung der Formel I und II zu erhalten, worin Y¹ und Y² Hydroxylschutzgruppen sind und, optional, entweder nachfolgendem Entfernen der Hydroxylschutzgruppen Y¹ und Y², oder Entfernen der Gruppen nach Erhalt des Sulfonylderivats und vor der Umsetzung mit der Jodidionenquelle, um die Verbindungen der Formel I oder II bereitzustellen, worin Y¹ und Y² beide Wasserstoff sind.

8. Verfahren gemäß Anspruch 7, wobei die 22-Hydroxyzwischenstufe durch Umsetzung eines Aldehyds der Formel IV oder V: 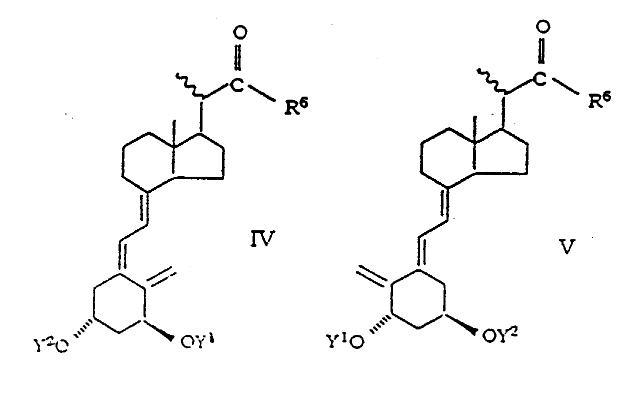 worin Y¹ und ² Hydroxylschutzgruppen sind und R⁶ Wasserstoff wiedergibt,
mit einem organometallischen Reagenz in einem inertem Lösungsmittel hergestellt wird.

9. Verfahren gemäß Anspruch 7, wobei die 22-Hydroxyzwischenstufe durch Umsetzen einer Verbindung der Formel IV oder V 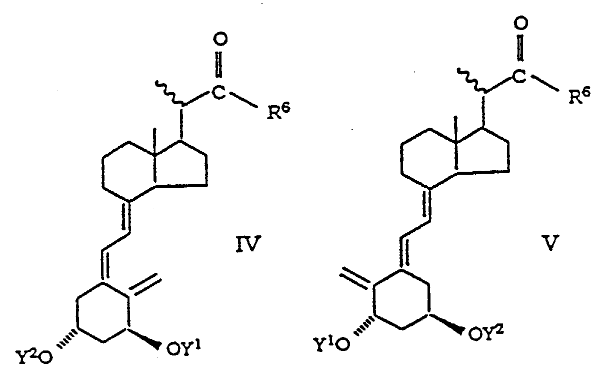 worin Y¹ und Y², die dieselben oder verschieden sein können, Wasserstoff oder eine Hydroxylschutzgruppe sind und R₆ O-Alkyl- oder O-Aryl wiedergibt,
mit einem organometallischen Reagenz zum Bereitstellen einer 22-Ketonverbindung der Formel IV oder V hergestellt wird, wobei R⁶ R wiedergibt wie in Anspruch 7 definiert und irgendwelche Hydroxylgruppen schützt, falls erforderlich; und Reduzieren der 22-Oxogruppe der 22-Ketonverbindung mit einem geeigneten Reduktionsmittel.

10. Verfahren gemäß Anspruch 7, wobei die 22-Hydroxyzwischenstufe durch Umsetzen eines diengeschützten Aldehydderivats der Formel III: 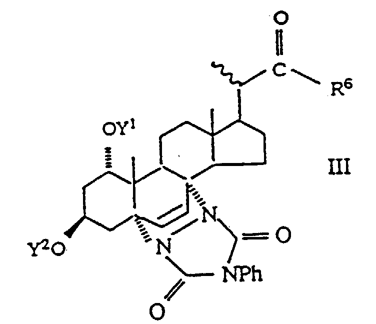 worin Y¹ und Y² Hydroxylschutzgruppen sind und R⁶ Wasserstoff wiedergibt, mit einem organometallischen Reagenz in einem inertem Lösungsmittel zur Herstellung eines Addukts der Formel VI 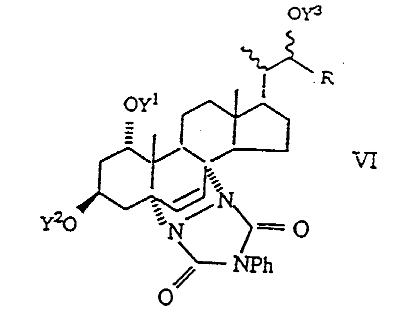 worin R wie in Anspruch 7 definiert ist und Y³ Wasserstoff, und Umwandeln des Addukts VI zu der erwünschten 22-Hydroxyzwischenstufe durch Entfernen der Triazolingruppe zur Bildung eines 5,7-Dien-Steroidintermediats, Unterwerfen des 5,7-Dien-Steroidintermediats einer Bestrahlung mit ultraviolettem Licht, um die entsprechende Provitamin D-Verbindung zu erhalten, und Isomerisieren der Provitamin D-Verbindung in einem Lösungsmittel bei einer Temperatur von 50°C bis 90°C hergestellt wird.

11. Verfahren gemäß Anspruch 7, wobei die 22-Hydroxyzwischenstufe hergestellt wird durch Umsetzen eines diengeschützten Esterderivats der Formel III 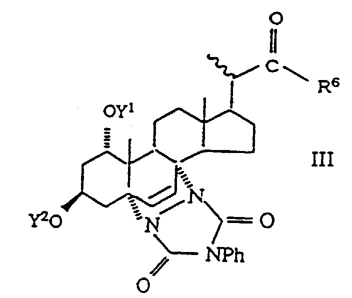 wobei Y¹ und Y² wie in Anspruch 9 definiert sind und R⁶ O-Alklyl oder O-Aryl wiedergibt, mit einem organometallischen Reagenz, um eine 22-Ketonverbindung der Formel III herzustellen, wobei R⁶ R wiedergibt wie in Anspruch 7 definiert, Schützen von irgendwelchen Hydroxylgruppen, falls erforderlich, und Reduzieren der 22-Oxogruppe der 22-Ketonverbindung mit einem geeigneten Reduktionsmittel, um ein Addukt der Formel VI herzustellen, wie in Anspruch 10 definiert, und konvertieren des Addukts VI zu der gewünschten 22-Hydroxyzwischenstufe durch Entfernen der Triazolingruppe zur Bildung eines 5,7-Dien-Steroidintermediats, Unterwerfen des 5,7-Dien-Steroidintermediats unter eine Bestrahlung mit ultraviolettem Licht, um die entsprechende Provitamin D-Verbindung zu erhalten, und Isomerisieren der Provitamin D-Verbindung in einem Lösungsmittel bei einer Temperatur von 50°C bis 90°C.

12. Verfahren gemäß einem der Ansprüche 7 bis 11, wobei das organometallische Reagenz ein Alkylmagnesiumhalogenid mit der Struktur RMgX ist oder ein Alkyllithium mit der Struktur RLi, worin R wie in Anspruch 7 definiert ist und X ein Halogen.

13. Verfahren gemäß einem der Ansprüche 7 bis 12, wobei das Jodid ein Tetraalkylammoniumjodid, Alkalimetalljodid oder Ammoniumjodid oder eine Mischung davon ist.

14. Verfahren gemäß einem der Ansprüche 7 bis 13, wobei der Schritt des Umsetzens des Sulfonylderivats mit der Jodidionenquelle in der Abwesenheit von Licht mit einer Wellenlänge von ungefähr 259 nm erfolgt.

15. Verfahren gemäß einem der Ansprüche 7 bis 14, wobei auch eine Zugabe von Quecksilber zu dem Reaktionsmedium während einer der Reaktionsschritte einschließt, bei denen Jodidverbindungen miteinbezogen sind.

16. Verfahren gemäß einem der Ansprüche 7 bis 15, wobei auch die Zugabe von Calciumkarbonat zu dem Reaktionsmedium während der nukleophilen Substitution des Sulfonyloxyderviats durch das Jodidion eingeschlossen ist.

17. Pharmazeutische Zusammensetzung, umfassend eine 22-Jodovitamin D-Verbindung wie in einem der Ansprüche 1 bis 6 beansprucht, oder eine durch ein wie in einem der Ansprüche 7 bis 16 beanspruchtes Verfahren erhaltene, zusammen mit einem pharmazeutisch verträglichen Träger.

18. Zusammensetzung gemäß Anspruch 17, wobei die 22-Jodoverbindung in Lösung in einem flüssigen, einnehmbaren und nicht-toxischen Vehikel vorliegt und die Lösung eingekapselt ist.

19. Zusammensetzung gemäß Anspruch 17, wobei dies eine Formulierung zur langsamen Freisetzung ist.

20. 22-Jodovitamin D₃-Verbindung wie in Anspruch 1 definiert, zur Verwendung bei der Behandlung von Osteoporose.

21. Verwendung gemäß Anspruch 20, wobei die 22-Jodovitamin D₃-Verbindung in einer Menge von 0,5 µg bis 500 µg pro Tag verabreicht wird.

22. Verwendung gemäß den Ansprüchen 20 oder 21 für senile Osteoporose.

23. Verwendung gemäß den Ansprüchen 20 oder 21 für postmenopausale Osteoporose.

24. Verwendung gemäß einem der Ansprüche 20 bis 23, wobei die Verbindung Frauen während und nach der Menopause verabreicht wird.

25. Verwendung gemäß einem der Ansprüche 20 bis 23, wobei die Verbindung Frauen vor dem Beginn der Menopause verabreicht wird.

26. Verwendung gemäß einem der Ansprüche 20 bis 25 für Inaktivitätsosteoporose (low bone turnover osteoporosis).

27. Verwendung gemäß einem der Ansprüche 20 bis 26, wobei die Verbindung täglich in unterteilten Dosierungen verabreicht wird.

## Revendications

1. Composé de type vitamine D ayant la structure I ou II présentées ci-dessous : 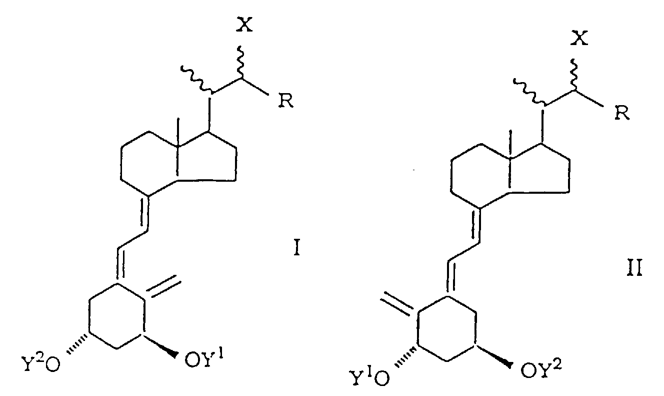 où X est un atome d'iode ou un atome d'iode radioactif, Y¹ et Y², identiques ou différents, représentent un atome d'hydrogène ou un groupe protecteur de fonctions hydroxyle, et R représente un atome d'hydrogène ou un groupe aryle, alkyle, hydroxyalkyle ou fluoroalkyle, ou le fragment de chaîne latérale suivant : 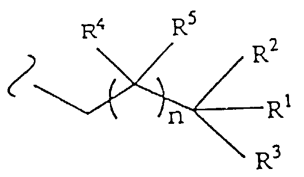 dans laquelle R¹ représente un atome d'hydrogène, un groupe hydroxy ou hydroxy protégé, R² et R³, identiques ou différents, représentent un groupe alkyle, hydroxyalkyle ou fluoroalkyle, ou, considérés conjointement, représentent le groupe -[CH₂]ₘ- où m est un nombre entier compris entre 2 et 5, R⁴ est un atome d'hydrogène ou de fluor ou un groupe hydroxy, O-acyle, alkyle, hydroxyalkyle ou fluoroalkyle, R⁵ représente un atome d'hydrogène ou de fluor ou un groupe alkyle, hydroxyalkyle ou fluoroalkyle et n est un nombre entier compris entre 1 et 5.

2. 22S- ou 22R-iodo-vitamine D₃.

3. 22S- ou 22R-iodo-25-hydroxyvitamine D₃.

4. 22S- ou 22R-iodo-1α-hydroxyvitamine D₃.

5. 22S- ou 22R-iodo-1α-25-dihydroxyvitamine D₃.

6. Composé de type vitamine D conforme à la revendication 1 dans lequel ledit atome d'iode radioactif est ¹²³I, ¹²⁵I, ¹²⁹I ou ¹³¹I.

7. Procédé de préparation d'un composé de type 22-iodo-vitamine D₃ comprenant les étapes consistant à faire réagir un intermédiaire hydroxylé en position 22 correspondant à la formule VII ou VIII : 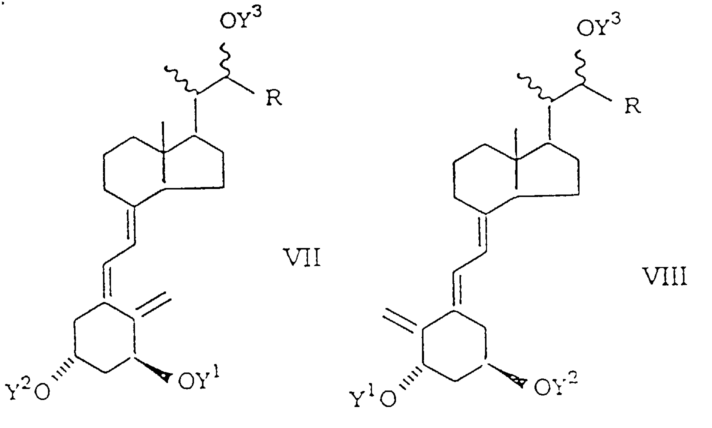 où Y¹ et Y², identiques ou différents, sont des groupes protecteurs de fonctions hydroxyle, Y³ est un atome d'hydrogène, R est défini comme dans la revendication 1, avec un halogénure d'alkylsulfonyle ou un halogénure d'arylsulfonyle dans un solvant pour obtenir le dérivé 22-O-alkylsulfonyle ou 22-O-arylsulfonyle correspondant, à faire réagir le dérivé sulfonyle avec une source d'ions iodure dans un solvant à une température comprise entre 0 °C et la température d'ébullition du solvant en présence de mercure et en absence de lumière d'une longueur d'onde voisine de la longueur d'onde d'absorption de la liaison carbone-iode, afin d'obtenir un composé 22-iodo de formule I et II où Y¹ et Y² représentent des groupes protecteurs de fonctions hydroxy, et, facultativement, à éliminer ensuite les groupes protecteurs de fonctions hydroxyle Y¹ et Y² ou à éliminer lesdits groupes après obtention du dérivé sulfonyle et avant réaction avec la source d'ions iodure, afin d'obtenir des composés de formule I ou II dans lesquels Y¹ et Y² représentent tous les deux un atome d'hydrogène.

8. Procédé conforme à la revendication 7 où l'on prépare l'intermédiaire hydroxylé en position 22 en faisant réagir un aldéhyde de formule IV ou V : 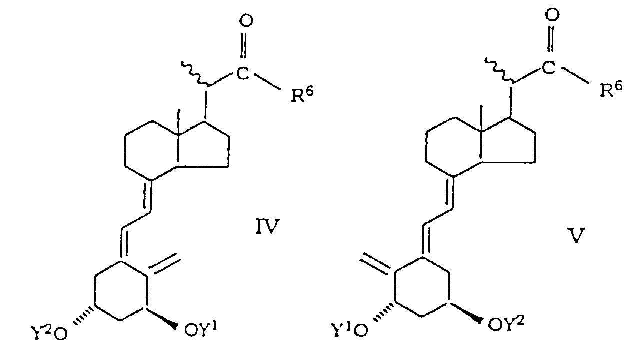 où Y¹ et Y² représentent des groupes protecteurs de fonctions hydroxyle et R⁶ représente un atome d'hydrogène, avec un réactif organométallique dans un solvant inerte.

9. Procédé conforme à la revendication 7 dans lequel on prépare l'intermédiaire hydroxylé en position 22 en faisant réagir un composé de formule IV ou V 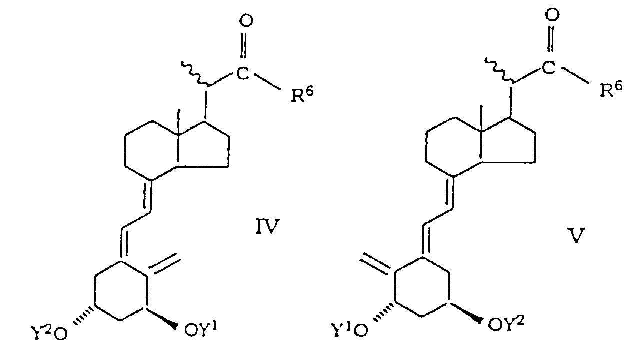 où Y¹ et Y², identiques ou différents, représentent un atome d'hydrogène ou un groupe protecteur de fonctions hydroxy et R⁶ représente un groupe O-alkyle ou O-aryle, avec un réactif organométallique afin d'obtenir un composé 22-cétone de formule IV ou V où R⁶ représente un groupe R tel qu'il est défini dans la revendication 7, en protégeant, si nécessaire, n'importe lequel des groupes hydroxy, et en réduisant le groupe oxo en position 22 dudit composé 22-cétone avec un agent réducteur approprié.

10. Procédé conforme à la revendication 7 dans lequel on prépare l'intermédiaire 22-hydroxy en faisant réagir un dérivé de type aldéhyde protégé par un diène et représenté par la formule III : 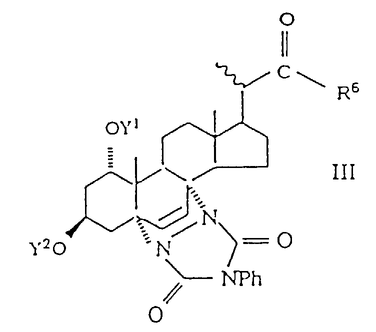 dans laquelle Y¹ et Y² représentent des groupes protecteurs de fonctions hydroxyle et R⁶ représente un atome d'hydrogène, avec un réactif organométallique dans un solvant inerte, ce qui donne un produit d'addition de formule VI 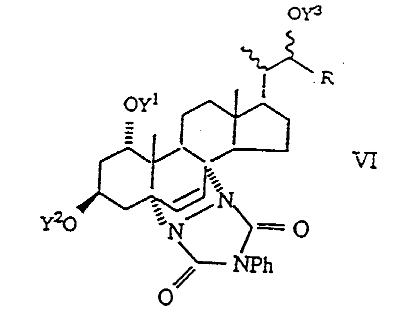 dans laquelle R est défini comme dans la revendication 7 et Y³ représente un atome d'hydrogène, en convertissant ledit produit d'addition VI en l'intermédiaire 22-hydroxy recherché par élimination du groupe triazoline ce qui donne un intermédiaire de type stéroïde 5,5-diène, en soumettant ledit intermédiaire de type stéroïde 5,7-diène à une irradiation par de la lumière ultraviolette afin d'obtenir la prévitamine D correspondante, et en isomérisant ladite prévitamine D dans un solvant à une température comprise entre 50 et 90 °C.

11. Procédé conforme à la revendication 7 dans lequel on prépare l'intermédiaire 22-hydroxy en faisant réagir un dérivé de type ester protégé par un diène et correspondant à la formule III : 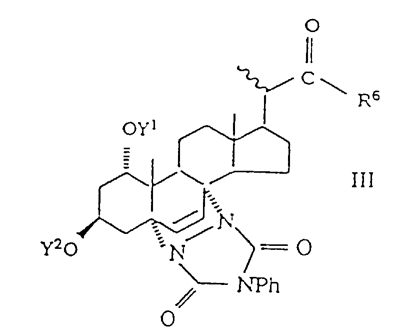 où Y¹ et Y² sont définis comme dans la revendication 9 et R⁶ représente un groupe O-alkyle ou O-aryle, avec un réactif organométallique, afin d'obtenir un composé 22-cétone de formule III où R⁶ représente un substituant R tel qu'il a été défini dans la revendication 7, en protégeant, si nécessaire, n'importe quel des groupes hydroxy et en réduisant le groupe oxo en position 22 dudit composé 22-cétone avec un agent réducteur approprié pour obtenir un produit d'addition de formule VI défini comme dans la revendication 10, en convertissant ledit produit d'addition VI en l'intermédiaire hydroxylé en position 22 recherché par élimination du groupe triazoline pour former un intermédiaire stéroïde 5,7-diène, en soumettant ledit intermédiaire stéroïde 5,7-diène à une irradiation par de la lumière ultraviolette afin d'obtenir la prévitamine D correspondante, et en isomérisant ladite prévitamine D dans un solvant à une température de 50 à 90 °C.

12. Procédé conforme à l'une quelconque des revendications 7 à 11 dans lequel le réactif organométallique est un halogénure d'alkylmagnésium de structure RMgX ou un alkyllithium de structure RLi où R est défini comme dans la revendication 7 et X représente un atome d'halogène.

13. Procédé conforme à l'une quelconque des revendications 7 à 12 dans lequel l'iodure est l'iodure de tétraalkylammonium, l'iodure d'un métal alcalin ou l'iodure d'ammonium ou un mélange de ceux-ci.

14. Procédé conforme à l'une quelconque des revendications 7 à 13 dans lequel l'étape consistant à faire réagir le dérivé sulfonyle avec la source d'ions iodure se fait en absence de lumière d'une longueur d'onde d'environ 259 nm.

15. Procédé conforme à l'une quelconque des revendications 7 à 14 comprenant également l'addition de mercure au milieu réactionnel pendant l'une quelconque des étapes réactionnelles mettant en jeu les composés de type iodure.

16. Procédé conforme à l'une quelconque des revendications 7 à 15 qui comprend également l'addition de carbonate de calcium au milieu réactionnel pendant la substitution nucléophile du dérivé sulfonyloxy par l'ion l'iodure.

17. Composition pharmaceutique comprenant un composé de type 22-iodo-vitamine D conforme à l'une quelconque des revendications 1 à 6 où un composé obtenu par un procédé conforme à l'une quelconque des revendications 7 à 16, en conjonction avec un excipient pharmaceutiquement acceptable.

18. Composition pharmaceutique conforme à la revendication 17 où le composé 22-iodo est en solution dans un véhicule liquide, pouvant être ingéré et non toxique et où la solution est encapsulée.

19. Composition conforme à la revendication 17, qui est une formulation à libération prolongée.

20. 22-iodo-vitamine D₃ conforme à la revendication 1 pour une utilisation dans le traitement de l'ostéoporose.

21. Composé conforme à la revendication 20 où la 22-iodo-vitamine D₃ est administrée à raison de 0,5 µg à 500 µg par jour.

22. Utilisation conforme à la revendication 20 ou 21 pour le traitement de l'ostéoporose sénile.

23. Utilisation conforme à la revendication 20 ou 21 pour le traitement de l'ostéoporose postménopausique.

24. Utilisation conforme à l'une quelconque des revendications 20 à 23 où le composé est administré à des femmes pendant et après la ménopause.

25. Utilisation conforme à l'une quelconque des revendications 20 à 23 où le composé est administré à des femmes avant ou au début de la ménopause.

26. Utilisation conforme à l'une quelconque des revendications 20 à 25 pour le traitement de l'ostéoporose à renouvellement osseux lent.

27. Utilisation conforme à l'une quelconque des revendications 20 à 26 où le composé est administré quotidiennement en plusieurs prises.
